# EUROPEAN PATENT SPECIFICATION

(11) **EP 1 175 208 B1**
(45) Date of publication and mention of the grant of the patent: **10.08.2005**
(21) Application number: 00921131.9
(22) Date of filing: 12.04.2000
(51) Int. Cl.: A61K 31/085, A61K 31/365, A61K 35/78, A61P 25/28

(54) **COMPOSITION FOR PREVENTING OR TREATING DEMENTIA COMPRISING A HYDROXYCINNAMIC ACID DERIVATIVE OR AN EXTRACT OF A PLANT OF GENUS ANGELICAE CONTAINING THE SAME**
ZUBEREITUNG ZUR PROPHYLAXE ODER BEHANDLUNG DER DEMENZ-ERKRANKUNG ENTHALTEND EIN HYDROXYZIMTSÄUREDERIVAT ODER EINEN EXTRAKT EINER PFLANZE DER GATTUNG ANGELICA, DER DIESE SÄURE ENTHÄLT.
COMPOSITION DE PREVENTION OU DE TRAITEMENT DE LA DEMENCE CONTENANT UN DERIVE D'ACIDE HYDROXYCINNAMIQUE OU UN EXTRAIT D'UNE PLANTE DE L'ESPECE ANGELICAE CONTENANT LEDIT ACIDE

(30) Priority: 13.04.1999 KR 9913613; 16.06.1999 KR 9923347
(43) Date of publication of application: 30.01.2002
(73) Proprietor: Scigenic Co., Ltd., Seoul 135-090 (KR); Biosynergen, Inc., Chunchon-si, Kangwon-do (KR)
(72) Inventor: KIM, Yung Hi, Chuncheon-si, Kangwon-do 200-162 (KR); SONG, Dong Keun, Chuncheon-si, Kangwon-do 200-163 (KR); SUH, Hong Won, Chuncheon-si, Kangwon-do 200-163 (KR); HUH, Sung Oh, Chuncheon-si, Kangwon-do 200-161 (KR)
(74) Representative: Andrews, Timothy Stephen
(86) International application number: PCT/KR2000/000337
(87) International publication number: WO 2000/061131

(56) References cited:
- EP-A2- 0 906 761
- US-A- 3 068 148
- US-A- 4 865 847
- US-A- 5 466 452
- US-A- 5 589 182
- US-A- 5 621 009
- PATENT ABSTRACTS OF JAPAN vol. 013, no. 063 (C-568), 13 February 1989 (1989-02-13) & JP 63 255222 A (NIPPON PETROCHEM CO LTD), 21 October 1988 (1988-10-21)
- DATABASE WPI Section Ch, Week 199237 Derwent Publications Ltd., London, GB; Class B04, AN 1992-304662 XP002236019 & JP 04 210643 A (ZH KITSUEN KAGAKU KENKYU ZAIDAN), 31 July 1992 (1992-07-31)
- DATABASE WPI Section Ch, Week 200151 Derwent Publications Ltd., London, GB; Class B04, AN 2001-466038 XP002236020 & CN 1 165 694 A (LIAO X), 26 November 1997 (1997-11-26)
- DATABASE CA [Online] YANAHARA NOBORU ET AL: 'Chinese herbs for the treatment of dementia of alzheimer type', XP002950619 Database accession no. 121:220838 & DEMENTIA vol. 8, no. 3, OSAKA, pages 293 - 302

## Description

### FIELD OF THE INVENTION

The present invention relates to use in the preparation of a medicament or food composition for preventing or treating dementia of a hydroxycinnamic acid derivative of formula I, decursinol, a pharmaceutically acceptable salt thereof or an extract of a plant of genus *Angelicae* containing same: 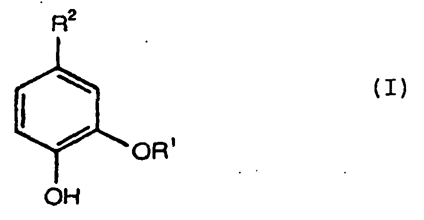 wherein,
R¹ is H or CH₃, and
R² is -CH=CHCOOH.

### BACKGROUND OF THE INVENTION

In recent years, an ever-increasing number of aged people have become afflicted by senile dementia, e.g., Alzheimer's disease.

It has been reported that accumulation of β-amyloid(Aβ₁₋₄₂) in the brain generates neurotoxicity, which constitutes one of the causes of Alzheimer's disease(Selkoe, Annu. Rev. Neurosci., 17, 489-517(1994)). Accordingly, numerous efforts have been made to develop a medicine for preventing or treating Alzheimer's disease by way of shielding brain cells from the toxicity of β-amyloid. However, no effective medicine has been developed.

US 5,621,009, US 3,068,148, US 4,865,847, JP 63-255222 and US 5,466,452 disclose various medical uses of caffeic acid, ferulic acid, isoferulic acid, chlorogenic acid, decursinol, and extracts of plants of the genus *Angelicae.*

JP 04-210643 discloses the ability of various phenolic compounds to stimulate nerve growth factor biosynthesis.

The present inventors have endeavored to develop a medicine for dementia and unexpectedly found that an extract of *Angelicae gigas* Nakai, which has been used in Korea for treating diseases of the heart, liver and spleen, has high activity in preventing or treating dementia.

### SUMMARY OF THE INVENTION

Accordingly, it is an object of the present invention to provide a pharmaceutical composition for preventing or treating dementia.

It is a further object of the present invention to provide a food composition for preventing or treating dementia.

In accordance with one aspect of the present invention, there is provided a use in the manufacture of a medicament for preventing or treating dementia of a hydroxycinnamic acid derivative of formula I or a pharmaceutically acceptable salt thereof: 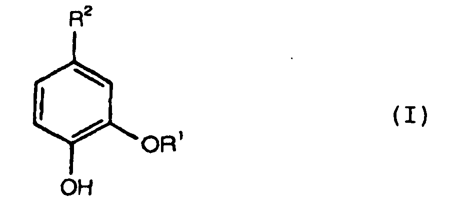 wherein,
R¹ is H or CH₃, and
R² is -CH=CHCOOH.

### BRIEF DESCRIPTION OF THE DRAWINGS

The above and other objects and features of the present invention will become apparent from the following description of the invention, when taken in conjunction with the accompanying drawings, which respectively show:
Figs. 1A and 1B: HPLC chromatograms confirming the presence of decursinol and ferulic acid in an extract of *Angelicae gigas* Nakai.
Fig. 2: the results of passive avoidance tests conducted employing mice administered with an extract of *Angelicae gigas* Nakai by changing the amount of the extract (Fig. 2A) and the period of administration(Fig. 2B);
Fig. 3: the results of passive avoidance tests conducted employing mice administered with ferulic acid by changing the amount of ferulic acid(Fig. 3A) and the period of administration(Fig. 3B);
Figs. 4A and 4B: the alternation behavior(%) and the number or arm entries, respectively, observed in Y-maze tests employing mice administered with ferulic acid by changing the period of administration;
Fig. 5: the results of passive avoidance tests conducted employing mice administered with ferulic acid or isoferulic acid;
Figs. 6a to 6c: the results of passive avoidance tests, the alternation behavior(%) and the number or arm entries observed in Y-maze tests conducted employing mice administered with various amounts of decursinol, respectively;
Figs. 7a and 7b: synergism between ferulic acid and decursinol observed in passive avoidance tests conducted employing mice administered with ferulic acid and decursinol;
Figs. 8a to 8f: micrographs of immunohistochemical staining of OX-42 in pyriform cortex and amygdaloid nucleus of a mouse, taken 1 day after the administration of β-42);
Figs. 9a to 9c: micrographs of immunohistochemical staining of choline acetyltransferase(ChAT) in septal nucleus of a mouse, taken 5 days after the administration of β-amyloid(1-42);
Fig. 10: the inhibitory effect of ferulic acid on β-amyloid(1-42)-induced leakage of lactate dehydrogenase(LDH) from the primary mouse cortical culture.

### DETAILED DESCRIPTION OF THE INVENTION

The present invention provides a use in the manufacture of a medicament for preventing or treating dementia of a hydroxycinnamic acid derivative of formula I or a pharmaceutically acceptable salt thereof: 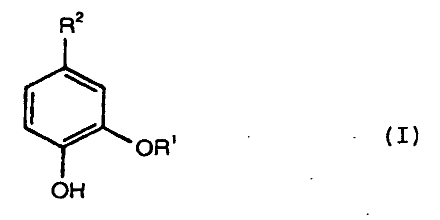 wherein,
R¹ is H or CH₃, and
R² is -CH=CHCOOH.

Exemplary hydroxycinnamic acid derivative of formula I includes ferulic acid and caffeic acid.

The present invention further provides a use in the preparation of a medicament for preventing or treating dementia of decursinol or a pharmaceutically acceptable salt thereof.

The ferulic acid and decursinol can be isolated from a plant of genus *Angelicae* according to the method described in "*Methods in New Drugs Development from Traditional Medicinal Materials*" (published by Seoul National University Natural Products Research Institute, Korea), or synthesized according to the method described in Merck Index. Further, ferulic acid is commercially available and other hydroxycinnamic acid derivatives can be prepared from ferulic acid by a simple process known in the art. In addition, they can be converted into various types of pharmaceutically acceptable salts, e.g., inorganic salts such as sodium, potassium, magnesium and calcium salts, and organic salts derived using angelic acid, lysine, ethanolamine, N,N'-dibenzylethylenediamine or α-tocopherol, or esters("oryzanol") derived using triterpene alcohol or plant sterols, e.g., cycloartenol, in accordance with the conventional methods well known in the art.

Further, the present invention also provides a use in the preparation of a medicament for preventing or treating dementia of an extract of a plant of genus *Angelicae* containing a hydroxycinnamic acid derivative of formula I as defined in claim 1 or decursinol. Examples of the plant of genus *Angelicae* useful in the present invention include *Angelicae gigas* Nakai, *Angelica acutiloba* Kitagawa and *Angelica sinensis* Diels.

The extract for use in the present invention may be prepared by any of the conventional methods using suitable solvents such as alcohols. For instance, a lower alcohol such as methanol and ethanol, preferably, 80 % methanol, is added to the root of a plant of genus *Angelicae* and the mixture is allowed to stand at a temperature ranging from 15 to 80°C, preferably, 30 to 55°C, for a period ranging from 15 minutes to 48 hours, preferably, 30 minutes to 12 hours to obtain an extract. The resulting extract comprises ferulic acid and decursinol in amounts ranging from 0.01 to 0.9 % by weight and 0.1 to 10 % by weight, respectively, based on the total weight of the extract. Further, the extract may also be prepared by employing an organic solvent, e.g., acetone, chloroform and methylene chloride, and may be processed into a powder by drying under a reduced pressure.

The hydroxycinnamic acid derivative of formula I, decursinol or an extract of a plant of genus *Angelicae* containing same exert a preventive or treating effect on dementia in a mouse Alzheimer's disease model: Administration of the composition is effective in preventing or treating memory impairment of a mouse induced by injecting which is established by administering β-amyloid(1-42) directly into the cerebral ventricle of the mouse.

The results of immunohistochemical staining to examine histological changes induced by injecting β-amyloid(1-42) into the cerebral ventricle of a control mouse show that OX-42, a marker of microglia, increases temporarily in the cerebral cortex and then returns to a normal state. Further, it is also observed that the level of choline acetyltransferase(ChAT), an enzyme synthesizing acetylcholine, decreases when β-amyloid(1-42) is administered.

In contrast, for a mouse which has been previously administered with the hydroxycinnamic acid derivative of formula I, decursinol or an extract of a plant of genus *Angelicae* before the administration of β-amyloid(1-42), the OX-42 level is much lower as compared with the control. Further, the decrease in the ChAT concentration observed with the control is not detected in the mouse administered with the inventive composition.

Thus, the hydroxycinnamic acid derivative of formula I, decursinol or an extract of a plant of genus *Angelicae* prevents or treats dementia through the protection of brain tissues by suppressing the activity of microglia which plays an important role in the induction of neurotoxicity by β-amyloid(1-42) and by preventing reduction of ChAT by β-amyloid(1-42).

Moreover, in spite of their potent efficacies, the hydroxycinnamic acid derivative of formula I, decursinol and an extract of a plant of genus *Angelicae* show little toxicity in toxicity tests using rats and exert no adverse effects on the liver function.

A pharmaceutical composition for preventing or treating dementia can be prepared by mixing the hydroxycinnamic acid derivative of formula I, decursinol or an extract of a plant of genus *Angelicae* with a pharmaceutically acceptable excipient or carrier, or by diluting it with a pharmaceutically acceptable diluent in accordance with any of the conventional procedures. Examples of suitable carriers, excipients, and diluents are lactose, dextrose, sucrose, sorbitol, mannitol, xylitol, erythritol, maltitol, starches, gum acacia, alginates, gelatin, calcium phosphate, calcium silicate, cellulose, methylcellulose, microcrystalline cellulose, polyvinylpyrrolidone, water, methylhydroxy-benzoates, propylhydroxybenzoates, talc, magnesium stearate and mineral oil. The formulations may additionally include fillers, anti-agglutinating agents, lubricating agents, wetting agents, flavors, emulsifiers, preservatives and the like. The compositions of the present invention may be formulated so as to provide quick, sustained or delayed release of the active ingredient after their administration to a mammal by employing any of the procedures well known in the art. Thus, the formulations may be in the form of a tablet, pill, powder, sachet, elixir, suspension, emulsion, solution, syrup, aerosol, soft and hard gelatin capsules, sterile injectable solution, sterile packaged powder and the like.

The pharmaceutical composition relating to the present invention can be administered via various routes including oral, transdermal, subcutaneous, intravenous and intramuscular introduction. Typical daily doses of the hydroxycinnamic acid derivative of formula I, decursinol and an extract of a plant of genus *Angelicae* may range from about 0.5 to 50 mg/kg body weight, 0.3 to 30 mg/kg body weight and 5 to 500 mg/kg body weight, respectively, and they can be administered in a single dose or in divided doses. However, it should be understood that the amount of the active ingredient actually administered ought to be determined in light of various relevant factors including the chosen route of administration, the age, sex and body weight of the individual patient, and the severity of the patient's symptom; and, therefore, the above dose should not be intended to limit the scope of the invention in any way.

On the other hand, the hydroxycinnamic acid derivative of formula I, decursinol or an extract of a plant of genus *Angelicae* can be incorporated in foods, as an additive or a dietary supplement, for the purpose of preventing or treating dementia. Accordingly, the present invention also provides the use in the preparation of a food composition for preventing or treating dementia of the hydroxycinnamic acid derivative of formula I, decursinol or an extract of a plant of genus *Angelicae* containing same. The foods may include various foodstuffs; beverages; gums; teas; vitamin complexes; and health foods.

In order to prepare the foods having preventive or treating activity on dementia, the hydroxycinnamic acid derivative of formula I, decursinol or an extract of a plant of genus *Angelicae* may be admixed with the raw materials during the preparation of foods or added to cooked foods. In this case, the content of the hydroxycinnamic acid derivative of formula I, decursinol and an extract of a plant of genus *Angelicae* in a food may range from 0.05 to 10 % by weight, 0.05 to 10 % by weight and 1 to 40 % by weight, respectively.

The following Examples are intended to further illustrate the present invention without limiting its scope.

Further, percentages given below for solid in solid mixture, liquid in liquid, and solid in liquid are on a wt/wt, vol/vol and wt/vol basis, respectively, and all the reactions were carried out at room temperature, unless specifically indicated otherwise.

### Reference Example 1: Injection into Cerebral Ventricle

The administration of β-amyloid(1-42) to a mouse was carried out in accordance with the method described by Laursen & Belknap (*J. Pharmacol. Methods, 16*, 355-357(1986)). Specifically, 5 µl of phosphate-buffered saline containing 1.85 µg of β-amyloid(1-42) was put in a 50 µl Hamilton syringe fitted with a 26 gauge needle, the tip of the needle was inserted into the bregma of the mouse and then the β-amyloid(1-42) solution was administered thereto. Each mouse of a control group received an equal amount of β-amyloid(42-1) in place of β-amyloid(1-42). A passive avoidance test was carried out at days 1-2(day 1: training, day 2: test) and a Y-maze test, at days 3-4 (day 3: training, day 4: test) after the administration.

### Reference Example 2: Passive Avoidance Test

In order to examine the learning and memory-retention ability of a mouse, a passive avoidance test was carried out in accordance with the method described in Song et al., *J. Neurochem*., *71*, 875-878(1998), as follows.

A passive avoidance case equipped with a light room and a dark room was prepared, the floor of the dark room being designed to deliver an electrical shock to a test animal. First, a mouse was put in the light room and, upon entering the dark room, it received an electrical shock at 0.25 mA for 1 sec. Twenty-four hours after the training, the mouse was put in the light room again and the time it took to enter the dark room was measured as a passive avoidance time. The maximum time was set at 300 sec., i.e., in case when the mouse took more than 300 sec. to enter the dark room, the passive avoidance time was determined to be 300 sec.

### Reference Example 3: Y-maze Test

Spontaneous alternation behavior of a mouse was examined by a Y-maze test in accordance with the method described in Starter et al., *Psychopharmacology, 94*, 491-495(1988) as follows.

The Y-maze consisted of three arms shaped like Y. A test mouse was placed in one of the arms such that it faced the arm's terminal and allowed to roan freely through the three arms for 8 hours. The number of alternation was determined by counting the number of occasions the mouse passed the three arms consecutively. Spontaneous alternation behavior was determined as the percentage of the alternation number based on the total number of arm entries.

### Example 1: Preparation of an extract of a plant of genus Angelicae

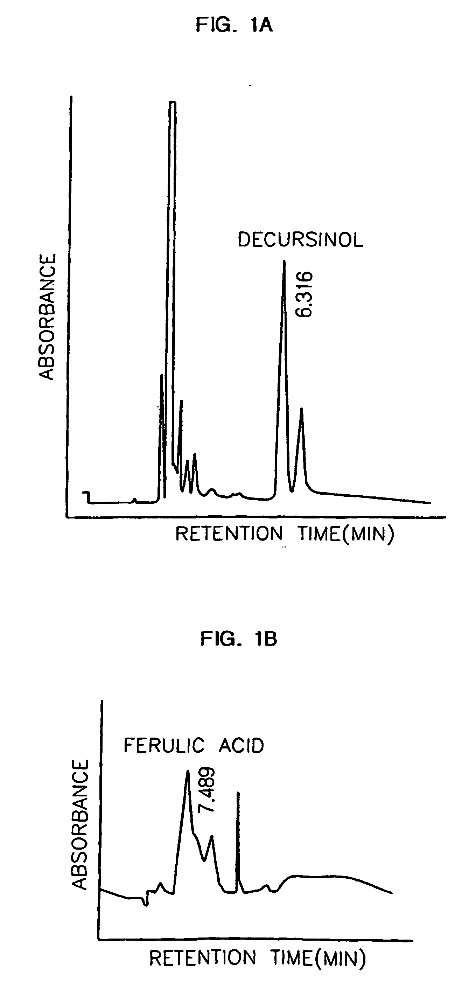

2 L of 80 % methanol was added to 1 kg of chopped root of *Angelicae gigas* Nakai and the mixture was heated at 40°C for 2 hours to obtain an extract. The extraction procedure was repeated twice. The extract solution were combined, and methanol and moisture was evaporated therefrom by employing a reduced-pressure reflux condenser. Then, the resulting extract was dried thoroughly at a reduced pressure to obtain 290 g of the extract of *Angelicae gigas* Nakai. HPLC analyses revealed that the extract contained decursinol and ferulic acid in amounts of 0.5 wt% and 0.07 wt%, respectively(Figs. 1A and 1B), the HPLC analyses being carried out under the following conditions:

### Decursinol

Column: Si60(7 µm, 250 mm × 4 mm); Solvent: n-hexane:EtoAc=1:1; Flow rate: 1.0 ml/min.; Detector: UV 340 nm

### Ferulic acid

Column: RP-18(7 µm, 250 nm × 4 mm); Solvent: MeOH:water=1:3.5; Flow rate: 1.0 ml/min.; Detector: UV 258 nm.

The above procedure was repeated to obtain extracts from *Angelica acutiloba* Kitagawa and *Angelica sinensis* Diels and it was confirmed by HPLC analysis that the resulting extracts also contained ferulic acid and decursinol.

### Example 2: Effect of the Extract of Angelicae gigas Nakai on the Prevention of Dementia

Forty 4- to 5- week old mice each weighing 20 to 25 g were divided into four groups of equal number. The *Angelicae gigas* Nakai extract prepared in Example 1 was dissolved in water to concentrations of 0.08 %(w/v) and 0.1 %(w/v), respectively, and the mice of two groups were provided with the resulting solutions instead of water for 4 weeks at a daily dose of 8 ml of the solution/mouse. The mice of the other two groups were provided with normal drinking water for 4 weeks. Thereafter, the mice of one of the two normal drinking water groups were administered into their cerebral ventricle with 1.85 µg of β-amyloid(42-1) ("control group") and the mice of the remaining three groups, with β-amyloid(1-42)(Aβ₁₋₄₂), as in Reference Example 1. Then, a passive avoidance test was carried out for each group of mice as in Reference Example 2 and the data attained were averaged for each group.

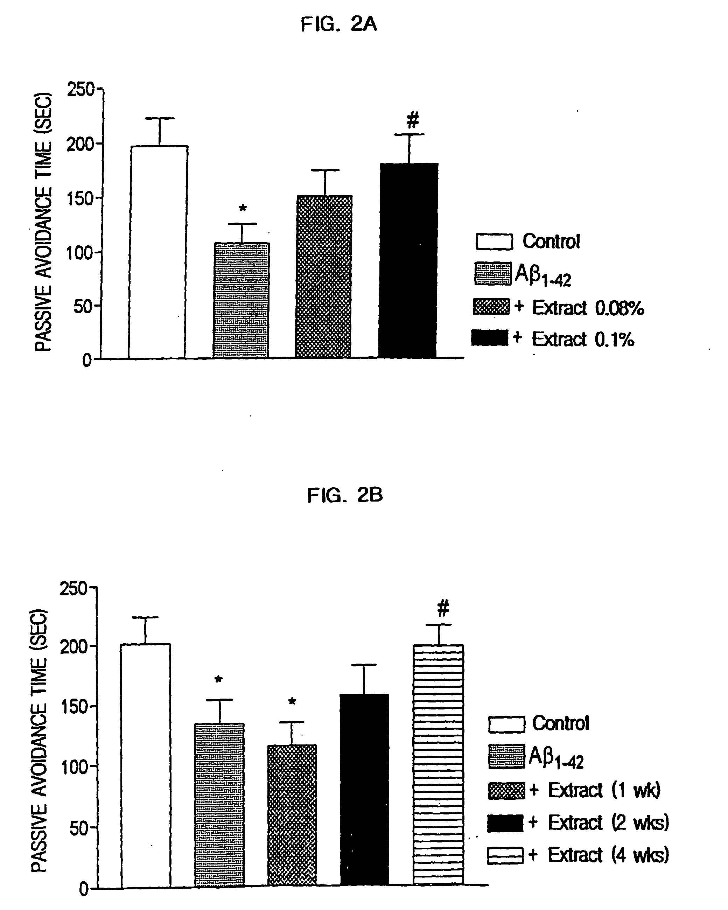

As shown in Fig. 2A, the passive avoidance response time was significantly lower for the group administered with β-amyloid(1-42) only, as compared with the control group. However, much improved passive avoidance response times were observed with the groups administered with 0.08 %(w/v) and 0.1 %(w/v) of the *Angelicae gigas* Nakai extract, respectively, as compared with the β-amyloid(1-42) group.

The same procedure as above was repeated by varying the administration period of 0.1 %(wjv) of the *Angelicae gigas* Nakai extract from 1 to 2 to 4 weeks. The result in Fig. 2B shows that improvement in the passive avoidance response time becomes evident when the administration of the *Angelicae gigas* Nakai extract is continued for 2 weeks or longer.

### Example 3: Effect of Ferulic Acid on Prevention of Dementia

The effect of ferulic acid, a component of the *Angelicae gigas* Nakai extract, on the prevention of dementia was examined as follows.

Fifty 4- to 5-week old mice each weighing 20 to 25 g were divided into five groups by equal number. Ferulic acid was dissolved in water to concentrations of 0.002 %(w/v), 0.004 %(w/v) and 0.006 %(w/v), respectively, and the mice of three groups were provided with the resulting solutions instead of water for 4 weeks at a daily dose of 8 ml of the solution/mouse. The mice of the other two groups were provided with normal drinking water for 4 weeks. Thereafter, the mice of one of two normal drinking water groups were administered into their cerebral ventricle with 1.85 µg of β-amyloid(42-1) ("control group") and the mice of the remaining four groups, with β-amyloid(1-42)(Aβ₁₋₄₂), as in Reference Example 1. Then, a passive avoidance test was carried out for each group of mice as in Reference Example 2 and the data attained were averaged for each group.

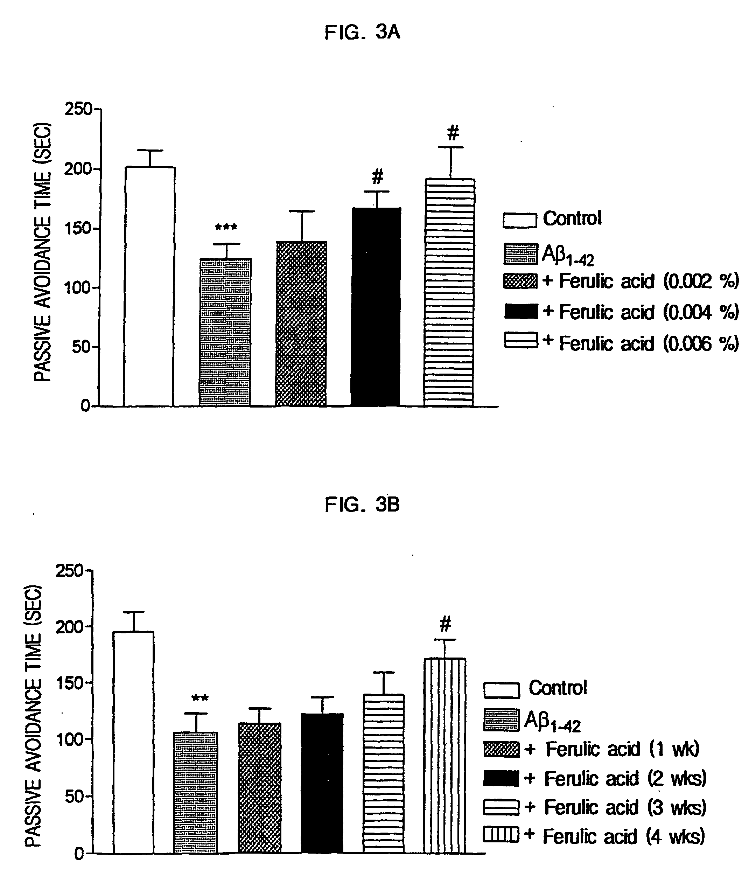

As shown in Fig. 3A, the passive avoidance response time was significantly lower for the group administered with β-amyloid(1-42) only, as compared with the control group. However, the decrease in the passive avoidance response time of the groups preventively administered with ferulic acid for 4 weeks was prevented dose-dependently.

Meanwhile, the above procedure was repeated by varying the period of administration of 0.006 %(w/v) of ferulic acid among 1, 2, 3 and 4 weeks. The result in Fig. 3B shows that the passive avoidance response time becomes increasingly higher with the period of administration.

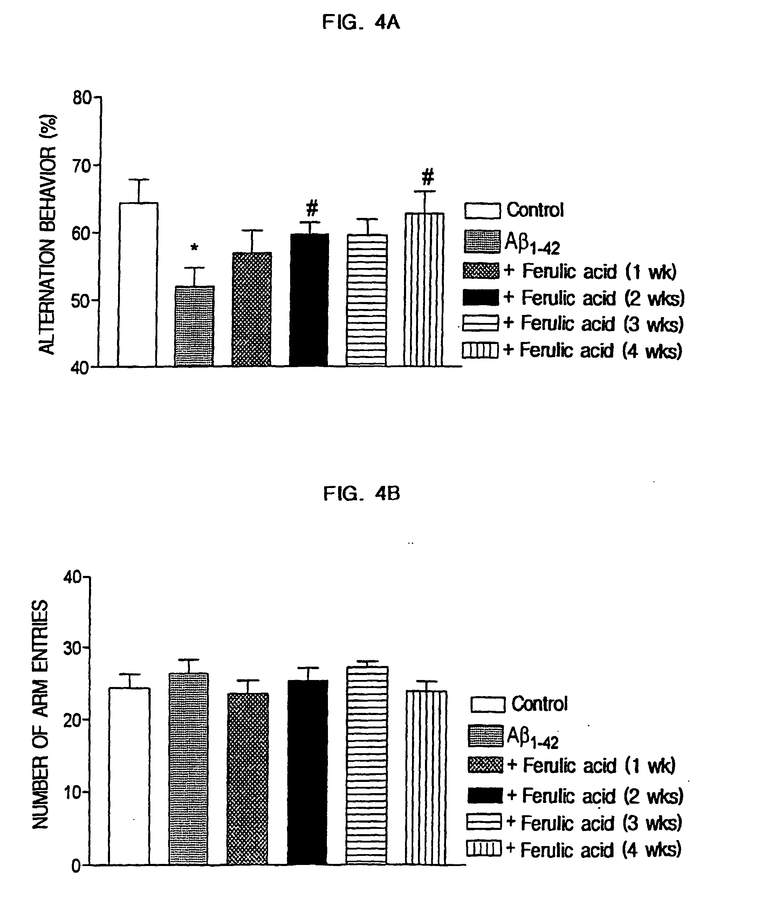

Further, Y-maze tests were carried out employing the above-prepared mice as in Reference Example 3. The result in Fig. 4A shows that, as compared with the group administered with β-amyloid(1-42) only, the alternation behavior(%) of the groups administered with ferulic acid was higher and increased in proportion to the period of administration. However, as shown in Fig. 4B, the number of arm entries, which represents a measure of spontaneous movement, is nearly the same for all groups. This result demonstrates that ferulic acid exerts therapeutic influence on the memory, rather than on movement.

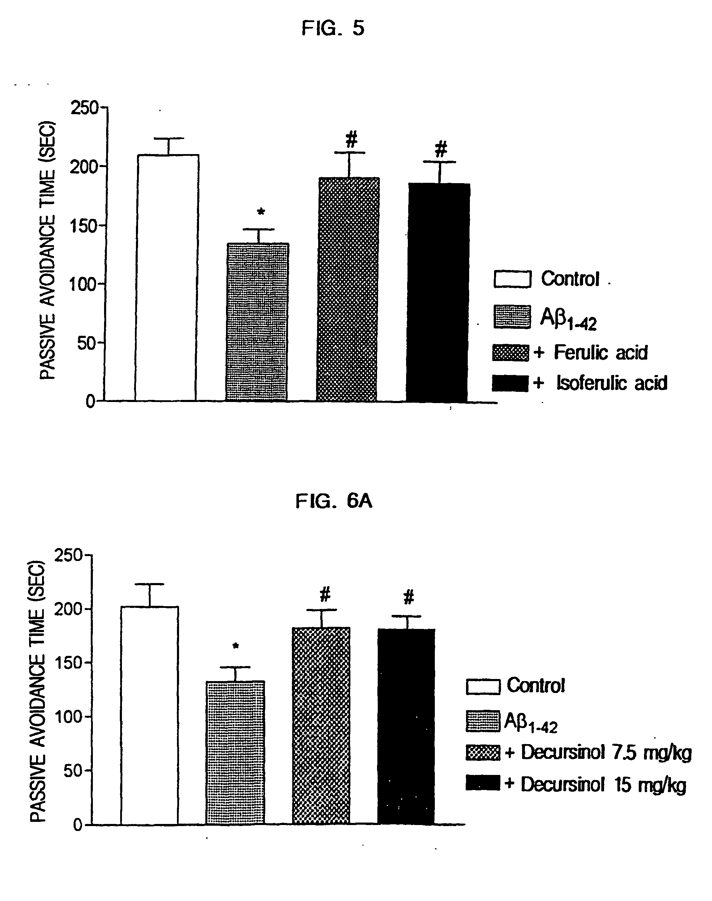

Passive avoidance tests were also conducted as above using mice administered with 0.006 %(w/v) of ferulic acid or isoferulic acid for 4 weeks. The result in Fig. 5 shows that the passive avoidance response time observed for the isoferulic acid group was similar to that of the ferulic acid group, demonstrating that isoferulic acid is as effective as ferulic acid in preventing dementia.

### Example 4: Effect of Decursinol on Prevention of Dementia

The effect of decursinol, a component of the *Angelicae gigas* Nakai extract, on the prevention of dementia was examined as follows.

Forty 4- to 5-week old mice each weighing 20 to 25 g were divided into four groups of equal number. Decursinol was dissolved in corn oil, mixed with a mouse fodder, and the mice of two groups were provided with the resulting fodder for 4 weeks at a daily dose of 7.5 mg decursinol/kg or 15 mg decursinol/kg. The mice of the other two groups were provided with a normal mouse fodder containing an equal amount of corn oil as above for 4 weeks. Thereafter, the mice of one of the two normal fodder groups were administered, into their cerebral ventricle, with 1.85 µg of β-amyloid(42-1) ("control group") and the mice of the remaining three groups, with β-amyloid(1-42)(Aβ₁₋₄₂), as in Reference Example 1. Then, a passive avoidance test and a Y-maze test were carried out for each group of mice as in Reference Examples 2 and 3, respectively, and the data attained were averaged for each group.

As shown in Fig. 6A, the decrease in the passive avoidance response time due to the administration of β-amyloid(1-42) was prevented in the groups preventively administered with decursinol.

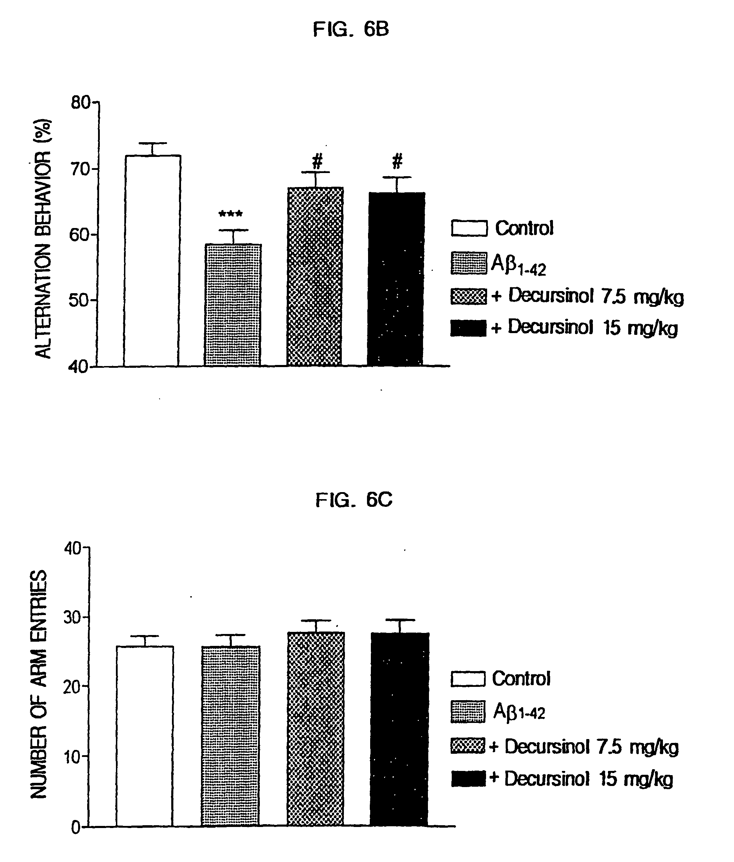

Figs. 6B and 6C show that, as compared with the group administered with β-amyloid(1-42) only, the alternation behavior(%) of the groups administered with decursinol was higher and increased dose-dependently. However, the number of arm entries, which represents a measure of spontaneous movement, is nearly the same for all groups. These results demonstrate that decursinol exerts therapeutic influence on the memory, rather than on movement.

In order to confirm the synergism between ferulic acid and decursinol, a passive avoidance test was carried out as in Reference Example 2 using mice administered with 0.002 %(w/v) of ferulic acid, 2 mg/kg of decursinol or a mixture thereof as above. Further, a passive avoidance test was also carried out using mice administered with 0.003 %(w/v) of ferulic acid, 3 mg/kg of decursinol or a mixture thereof. In the two experiments, a group of mice administered with 0.006 %(w/v) of ferulic acid was employed as a comparative group.

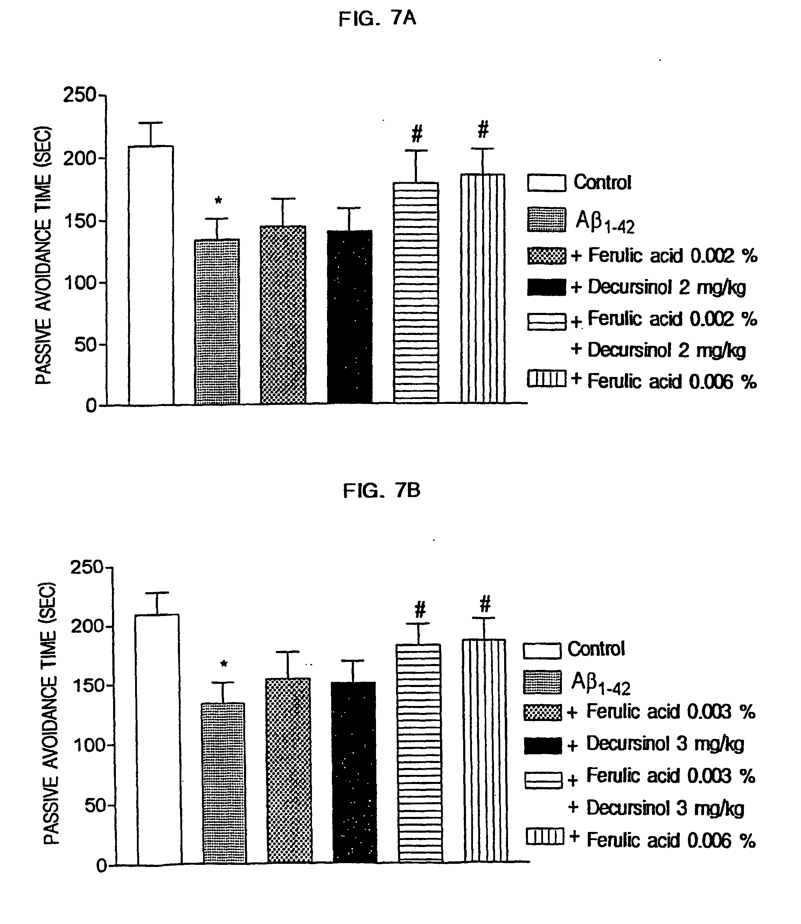

The results in Figs. 7A and 7B show that a mixture of ferulic acid and decursinol, each of them being in a low amount having no dementia-preventive effect, exhibit a significant preventive effect. This result demonstrates the synergism between ferulic acid and decursinol.

### Example 5: Examination of the Activity of Ferulic Acid Protecting Brain Cells

### (1) Immunohistochemical staining of OX-42 at one day after the administration of β-amyloid

4- to 5-week old mice each weighing 20 to 25 g was divided into three groups. Ferulic acid was dissolved in water to a concentration of 0.006 %(w/v), and the mice of one group were provided with the resulting solution instead of water for 4 weeks. The mice of other two groups were provided with normal drinking water for 4 weeks. Thereafter, the mice of one of two normal drinking water groups were administered, into their cerebral ventricle, with 1.85 µg of β-amyloid(42-1) ("control group") and the mice of the remaining two groups, with β-amyloid(1-42)(Aβ₁₋₄₂), as in Reference Example 1

One day after the administration of β-amyloid, each of the mice was anesthetized with sodium pentobarbital and its abdomen was incised. The mouse was fixed by passing 4% paraformaldehyde through its left ventricle and its brain was excised.

In accordance with the method described in Cho *et al*., *J. Comp. Neurol*., *369:* 264-276(1996), a section was prepared from the excised brain and immunohistochemical staining was carried out in order to stain OX-2 in the pyriform cortex and the amygdaloid nucleus by employing 1:500 dilution of anti-OX-42 antibody(Halan(Sera-Lab)). Then, the section was observed with a microscope at magnifications of 200 and 400, respectively.

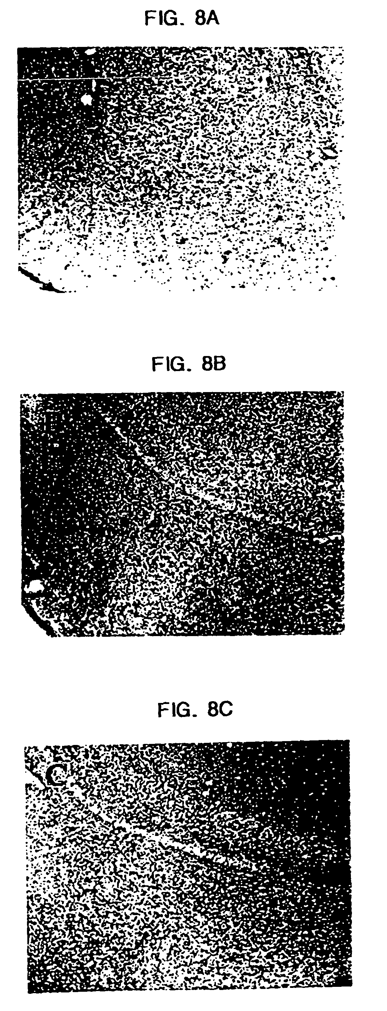

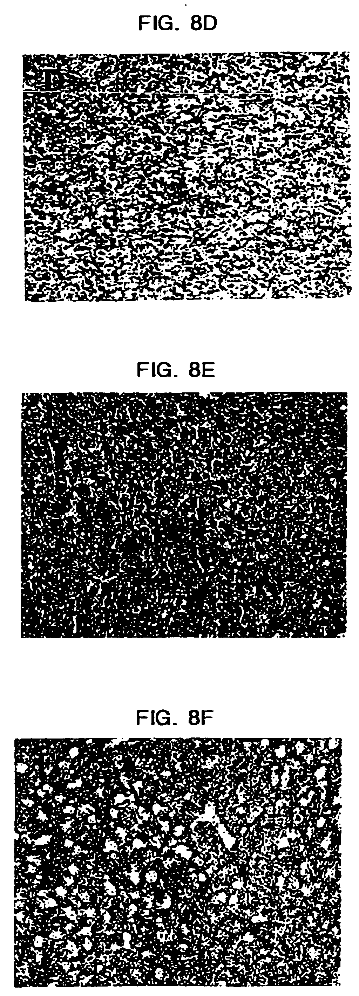

The result is shown in Figs. 8a to 8f, wherein Figs. 8a to 8c were taken at a magnification of 200, and Figs. 8d to 8f, at a magnification of 400. As compared with the control group(Figs. 8a and 8d), increased OX-42 was observed with the group administered with β-amyloid(1-42) only(Figs. 8b and 8e), while OX-42 is much less pronounced with the group administered with ferulic acid followed by β-amyloid(1-42)(Figs. 8c and 8f).

### (2) Immunohistochemical staining of ChAT 5 days after the administration of β-amyloid

Three groups of mice were administered with ferulic acid and/or β-amyloid in accordance with the method of (1). 5 days after the administration of β-amyloid, immunohistochemical staining was carried out according to the method of (1) except for employing 1:750 dilution of anti-choline-acetyltransferase(ChAT) antibody(Chemicon) in order to stain ChAT, an enzyme for acetylcholine synthesis, in the septal nucleus wherein acetylcholine neurons are distributed.

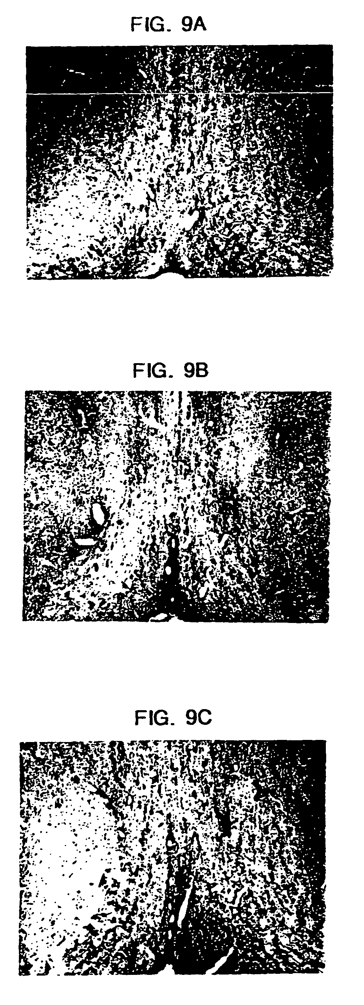

The result is shown in Figs. 9a to 9c. As compared with the control group(Fig. 9a), decreased ChAT was observed with the group administered with β-amyloid(1-42) only(Fig. 9b) while the decrease was suppressed in the group administered with ferulic acid followed by β-amyloid(1-42)(Fig. 9c).

### Example 6: Effect of ferulic acid in inhibiting the damage of neurons

In accordance with the method of Wie *et al*. (*Neurosci. Lett*., *225*: 93-96(1997)), glia was cultured from cells of the cerebral cortex of a mouse and neurons of mouse cerebrum were cultured by employing the glia as a substrate.

The culture of mouse cerebral neurons was divided into three groups. Neurons of one group received no treatment(control group). Neurons of the other two groups were treated with 25 µM of β-amyloid(1-42), and one of these two groups were treated with 100 µM of ferulic acid 30 minutes before the administration of β-amyloid(1-42).

The degree of damage in neurons was determined by measuring the concentration of lactate dehydrogenase(LDH), which was released from the damaged or disrupted neurons into the culture medium, in accordance with the method of Wie et al. Samples of the culture medium were taken at 24 and 48 hours after the administration of β-amyloid(1-42), respectively, and their LDH concentrations were measured with a microplate reader.

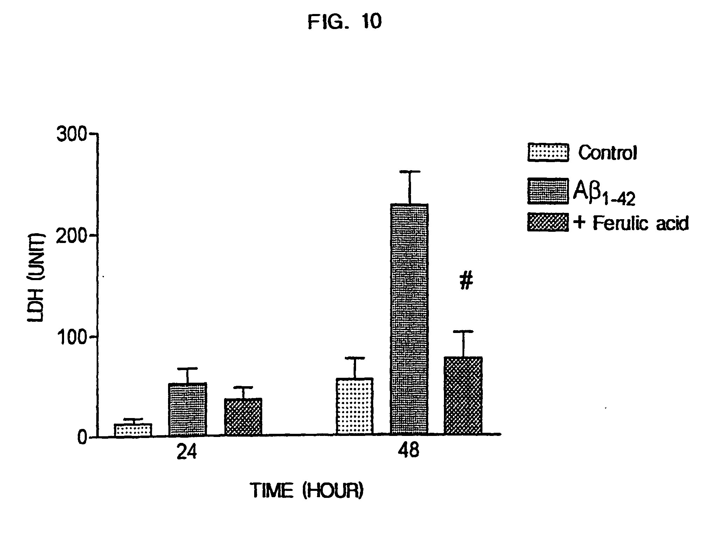

As shown in Fig. 10, as compared with the control group, the LDH concentration is significantly higher for the group administered with β-amyloid(1-42), while a much lower LDH concentration was observed with the group administered with ferulic acid followed by β-amyloid(1-42). This result demonstrates that ferulic acid inhibits the damage of neurons caused by β-amyloid(1-42).

### Example 7: Toxicity of the Extract of Angelica gigas Nakai

Forty(20 males and 20 females) 4 week-old Sprague-Dawley rats were bred for a week under a condition of temperature 22±3°C, relative humidity 50 ±10% and intensity of illumination 150-300 Lux. The rats were divided into four groups each consisting of 5 males and 5 females.

The extract of *Angelica gigas* Nakai prepared in Example 1 was dissolved in corn oil and administered orally to the rats of four groups at doses of 300, 1,000, 3000 and 10,000 mg/kg, respectively. The extract was administered once and the rats were observed for 7 days for signs of adverse effect or death. Further, on the 7th day, the rats were sacrificed and the internal organs were visually examined. The weight changes of the rats were recorded every day to examine the effect of the extract of *Angelica gigas* Nakai.

The result showed that LD₅₀ of the extract of *Angelica gigas* Nakai is 3,722 mg/kg for males and 2,804 mg/kg for females. The autopsy showed that the rats did not develop any pathological abnormality at a dose of 1,000 mg/kg or less. Further, no weight loss was observed during the 7-day test period in case of the rats administered with the extract at a dose of 1,000 mg/kg or less.

### Formulation Example

100 mg of the *Angelicae gigas* Nakai extract prepared in Example 1, 45 mg of milk calcium, 122 mg of microcrystalline cellulose, 15 mg of isoflavon, 2.5 mg of ginkgo extract, 2 mg of *Zizyphus jujuba* extract, 0.25 mg of vitamin B₁, 0.3 mg of vitamin B₂, 0.0025 mg of vitamin D₃ and 2.5 mg of magnesium stearate were mixed thoroughly and filled into a hard gelatin capsule to prepare a hard gelatin capsule formulation.

While the invention has been described with respect to the above specific embodiments, it should be recognized that various modifications and changes may be made to the invention by those skilled in the art which also fall within the scope of the invention as defined by the appended claims.

## Claims

1. Use in the manufacture of a medicament for preventing or treating dementia of a hydroxycinnamic acid derivative of formula I or a pharmaceutically acceptable salt thereof: 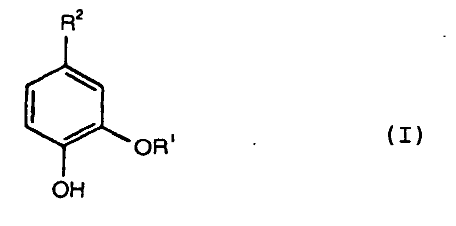 wherein,
R¹ is H or CH₃, and
R² is -CH=CHCOOH.

2. The use of claim 1, wherein the hydroxycinnamic acid derivative of formula I is ferulic acid.

3. The use of claim 1 or claim 2, wherein said medicament further comprises decursinol.

4. The use in the preparation of a medicament for preventing or treating dementia of decursinol or a pharmaceutically acceptable salt thereof.

5. The use in the preparation of a medicament for preventing or treating dementia of an extract of a plant of genus *Angelicae* containing a hydroxycinnamic acid derivative of formula I as defined in claim 1 or decursinol.

6. The use of claim 5 wherein the extract is prepared by extracting the root of a plant of genus *Angelicae* with a lower alcohol.

7. The use of claim 5 or 6 wherein the plant of genus *Angelicae* is selected from the group consisting of *Angelicae gigas* Nakai, *Angelica acutiloba* Kitagawa and *Angelica sinensis* Diels.

8. Use in the preparation of a food composition for preventing or treating dementia of a hydroxycinnamic acid derivative of formula I as defined in claim 1 or a pharmaceutically acceptable salt thereof.

9. The use of claim 8, wherein the hydroxycinnamic acid derivative of formula I is ferulic acid.

10. The use of claim 8 or claim 9, wherein said food composition further comprises decursinol.

11. The use in the preparation of a food composition for preventing or treating dementia of decursinol or a pharmaceutically acceptable salt thereof.

12. The use in the preparation of a food composition for preventing or treating dementia of an extract of a plant of genus *Angelicae* containing a hydroxycinnamic acid derivative of formula I as defined in claim 1 or decursinol.

13. The use of claim 12, wherein the extract is prepared by extracting the root of a plant of genus *Angelicae* with a lower alcohol.

14. The use of claim 12 or 13 wherein the plant of genus *Angelicae* is selected from the group consisting of *Angelicae gigas* Nakai, *Angelica acutiloba* Kitagawa and *Angelica sinensis* Diels.

## Patentansprüche

1. Verwendung eines Hydroxyzimtsäure-Derivats der Formel I oder eines pharmazeutisch verträglichen Salzes davon bei der Herstellung eines Arzneimittels zur Prävention oder Behandlung der Demenz: 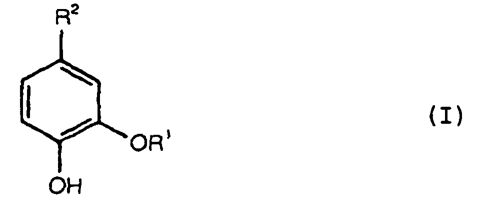 worin
R¹ für H oder CH₃ steht, und
R² für -CH=CHCOOH steht.

2. Verwendung nach Anspruch 1, worin das Hydroxyzimtsäure-Derivat der Formel I für Ferulsäure steht.

3. Verwendung nach Anspruch 1 oder 2, worin das Arzneimittel weiter Decursinol umfasst.

4. Verwendung von Decursinol oder eines pharmazeutisch verträglichen Salzes davon bei der Herstellung eines Arzneimittels zur Prävention oder Behandlung der Demenz.

5. Verwendung eines Extrakts einer Pflanze der Gattung *Angelicae,* enthaltend ein Hydroxyzimtsäure-Derivat der Formel I wie nach Anspruch 1 definiert oder Decursinol, bei der Herstellung eines Arzneimittels zur Prävention oder Behandlung der Demenz aus.

6. Verwendung nach Anspruch 5, worin der Extrakt durch Extrahieren der Wurzel einer Pflanze der Gattung *Angelicae* mit einem niederen Alkohol hergestellt wird.

7. Verwendung nach Anspruch 5 oder 6, worin die Pflanze der Gattung *Angelicae* aus der Gruppe ausgewählt ist, bestehend aus *Angelica gigas* Nakai, *Angelica acutiloba* Kitagawa und *Angelica sinensis* Diels.

8. Verwendung eines Hydroxyzimtsäure-Derivats der Formel I wie nach Anspruch 1 definiert oder eines pharmazeutisch verträglichen Salzes davon bei der Herstellung einer Nahrungsmittelzusammensetzung zur Prävention oder Behandlung der Demenz.

9. Verwendung nach Anspruch 8, worin das Hydroxyzimtsäure-Derivat der Formel I für Ferulsäure steht.

10. Verwendung nach Anspruch 8 oder 9, worin die Nahrungsmittelzusammensetzung weiter Decursinol umfasst.

11. Verwendung von Decursinol oder eines pharmazeutisch verträglichen Salzes davon bei der Herstellung einer Nahrungsmittelzusammensetzung zur Prävention oder Behandlung der Demenz.

12. Verwendung eines Extrakts einer Pflanze der Gattung *Angelicae,* enthaltend ein Hydroxyzimtsäure-Derivat der Formel I wie nach Anspruch 1 definiert oder Decursinol, bei der Herstellung einer Nahrungsmittelzusammensetzung zur Prävention oder Behandlung der Demenz.

13. Verwendung nach Anspruch 12, worin der Extrakt durch Extrahieren der Wurzel einer Pflanze der Gattung *Angelicae* mit einem niederen Alkohol hergestellt wird.

14. Verwendung nach Anspruch 12 oder 13, worin die Pflanze der Gattung *Angelicae* ausgewählt ist aus der Gruppe, bestehend aus *Angelica gigas* Nakai, *Angelica acutiloba* Kitagawa und *Angelica sinensis* Diels.

## Revendications

1. Utilisation dans la fabrication d'un médicament de prévention ou de traitement de la démence d'un dérivé de l'acide hydroxycinnamique de formule 1 ou d'un sel pharmaceutiquement acceptable de celui-ci: 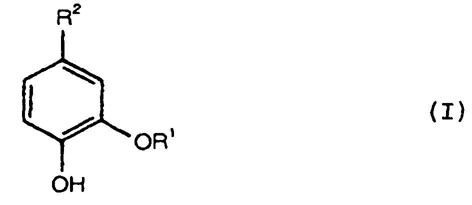 dans laquelle
R¹ est H ou CH₃, et
R² est -CH=CHCOOH.

2. Utilisation de la revendication 1, dans laquelle le dérivé de l'acide hydroxycinnamique de formule I est l'acide férulique.

3. Utilisation de la revendication 1 ou de la revendication 2, dans laquelle ledit médicament comprend en outre du decursinol.

4. Utilisation dans la préparation d'un médicament de prévention ou de traitement de la démence du decursinol ou d'un sel pharmaceutiquement acceptable de celui-ci.

5. Utilisation dans la préparation d'un médicament de prévention ou de traitement de la démence d'un extrait d'une plante du genre *Angelicae* contenant un dérivé de l'acide hydroxycinnamique de formule I comme défini dans la revendication 1 ou du decursinol.

6. Utilisation de la revendication 5 dans laquelle l'extrait est préparé en extrayant la racine d'une plante du genre *Angelicae* avec un alcool inférieur.

7. Utilisation de la revendication 5 ou 6 dans laquelle la plante du genre *Angelicae* est choisie dans le groupe comprenant *Angelicae gigas* Nakai, *Angelica acutiloba* Kitagawa et *Angelica sinensis* Diels.

8. Utilisation dans la fabrication d'une composition alimentaire de prévention ou de traitement de la démence d'un dérivé de l'acide hydroxycinnamique de formule I comme défini dans la revendication 1 ou d'un sel pharmaceutiquement acceptable de celui-ci.

9. Utilisation de la revendication 8, dans laquelle le dérivé de l'acide hydroxycinnamique de formule 1 est l'acide férulique.

10. Utilisation de la revendication 8 ou de la revendication 9, dans laquelle ladite composition alimentaire comprend en outre du decursinol.

11. Utilisation dans la préparation d'une composition alimentaire de prévention ou de traitement de la démence du decursinol ou d'un sel pharmaceutiquement acceptable de celui-ci.

12. Utilisation dans la préparation d'une composition alimentaire de prévention ou de traitement de la démence d'un extrait d'une plante du genre *Angelicae* contenant un dérivé de l'acide hydroxycinnamique de formule I comme défini dans la revendication 1 ou du decursinol.

13. Utilisation de la revendication 12 dans laquelle l'extrait est préparé en extrayant la racine d'une plante du genre *Angelicae* avec un alcool inférieur.

14. Utilisation de la revendication 12 ou 13 dans laquelle la plante du genre *Angelicae* est choisie dans le groupe comprenant *Angelicae gigas* Nakai, *Angelica acutiloba* Kitagawa et *Angelica sinensis* Diels.
